(19) **Europäisches Patentamt** | **European Patent Office** | **Office européen des brevets**

(11) **EP 4 664 399 A1**

(12) **EUROPEAN PATENT APPLICATION**

(43) Date of publication:
**17.12.2025 Bulletin 2025/51**

(21) Application number: **24465529.6**

(22) Date of filing: **10.06.2024**

(51) International Patent Classification (IPC):
**G06T 7/13** $^{(2017.01)}$ **G06T 7/162** $^{(2017.01)}$

(52) Cooperative Patent Classification (CPC):
**G06T 7/13; G06T 7/162;** G06T 2207/10121;
G06T 2207/20072; G06T 2207/30101

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG
91301 Forchheim (DE)**

(72) Inventors:
- **Neumann, Dominik
  91052 Erlangen (DE)**
- **Turcea, Alexandru
  105500 Busteni (RO)**

(74) Representative: **Siemens Healthineers
Patent Attorneys
Postfach 22 16 34
80506 München (DE)**

Remarks:
Amended claims in accordance with Rule 137(2)
EPC.

(54) **AGGREGATED REPRESENTATION OF A VESSEL STRUCTURE**

(57)     Graph representations (11) of a vessel structure (18) are received, each corresponding to a medical image (10) and comprising a plurality of nodes and respective spatial coordinates for each node, wherein the plurality of nodes is arranged according to at least one segment (12a, 12b). For each segment (12a, 12b) of a first graph representation (11), it is determined whether a second graph representation (11) of the plurality of graph representations (11) comprises a segment (12a, 12b) corresponding to the respective segment (12a, 12b) of the first graph representation (11) and, if it is found that this is not the case, the second graph representation (11) is augmented by adding an additional segment (12a, 12b) corresponding to the respective segment (12a, 12b) of the first graph representation (11). An aggregated representation of the vessel structure (18) is generated depending on the augmented second graph representation (11).

FIG 3

EP 4 664 399 A1

## Description

The present invention is directed to a computer-implemented method for generating at least one aggregated representation of a vessel structure, to a method for generating at least one aggregated representation of a vessel structure, to a data processing system for carrying out said computer-implemented method, to a medical imaging system comprising such a data processing system, and to a corresponding computer program product.

Medical imaging of vessel structures, also denoted as angiography, is widely used for assisting at medical interventions. In particular, invasive coronary X-Ray angiography is the method of choice for the assessment of coronary arteries and the diagnosis of coronary artery disease, CAD. Software tools enhanced by artificial intelligence, AI, components are slowly finding their entry into the cath labs to support interventional cardiologists in their decision making, potentially leading to improved efficiency, diagnostic accuracy, and reproducibility.

Over the last years, several modules for different tasks such as vessel detection, vessel segmentation, branch labeling, computation of the fractional flow reserve, FFR, or another hemodynamic characteristics, and so forth have been developed, many of them based on AI. Some of these modules have the advantage that their analysis is not isolated to a single frame within an angiography sequence, but instead it aggregates information across multiple frames, for example over a full heart cycle, which aims to get a better understanding of the true state of the anatomy and physiology of the patient's coronaries.

However, aggregation of information extracted from multiple frames poses major challenges. For example, it may happen that not all portions of the vessel structure can be extracted from each individual frame, for example due to the motion of the patient, the cardiac motion, changing angulation of the imaging device and so forth, which leads to inconsistencies in the representation of the vessel structure. This may reduce the reliability and/or accuracy of the subsequent tasks' results. It would therefore be desirable to have a more universal representation of a vessel structure over several frames of medical images.

It is an objective of the present invention to provide a more consistent representation of a vessel structure over several frames of medical images.

This objective is achieved by the subject matter of the independent claim. Further implementations and preferred embodiments are subject matter of the dependent claims.

The invention is based on the idea to generate at least one aggregated representation of a vessel structure based on respective graph representations of the same vessel structure depicted in medical images by attaching segments of a first graph representation to a second graph representation, in case the second graph representation does not comprise a respective segment already.

According to an aspect of the invention, a computer-implemented method for generating at least one aggregated representation of a vessel structure is provided. Therein, respective graph representations of the vessel structure are received. Therein, each graph representation of the received graph representations corresponds to a respective medical image of a sequence of consecutive medical images, each of the medical images depicting the vessel structure. Each of the graph representations comprises a plurality of nodes, in particular consists of the plurality of nodes and edges connecting the nodes, and respective spatial coordinates for each node of the respective plurality of nodes. Therein, the plurality of nodes is arranged according to at least one segment of the respective graph representation. For each first segment of the plurality of segments of a first graph representation of the plurality of graph representations, the following steps i) and ii) are carried out. Therein, segments of the plurality of segments of a first graph representation of the plurality of graph representations are denoted as first segments and segments of the plurality of segments of a second graph representation of the plurality of graph representations are denoted as second segments. It is noted that the plurality of graph representations is not necessarily ordered in a specific way and, in particular, the first graph representation is not necessarily an initial graph representation according to such an order and, in particular, the second graph representation does not necessarily follow the first graph representation according to such an order.

In step i), it is determined whether the second graph representation of the plurality of graph representations comprises a second segment corresponding to the respective first segment of the first graph representation, in particular depending on the spatial coordinates of the nodes of the first segment and the nodes of the second graph representation. In step ii), the second graph representation is augmented if it is found that the second graph representation does not comprise a second segment corresponding to the respective first segment of the first graph representation in step i). Therein, augmenting the second graph representation comprises adding an additional second segment corresponding to the respective first segment of the first graph representation to the second graph representation, for example depending on the spatial coordinates of the nodes of the first segment and the nodes of the second graph representation. After steps i) and ii) have been carried out for all first segments of the first graph representation, the at least one aggregated representation is generated depending on the augmented second graph representation.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for generating at least one aggregated representation of a vessel structure, which is not purely computer-implemented, is obtained by including respective steps of generating the sequence of consecutive medical images, in particular by the imaging device.

The imaging device may be any medical imaging device, which is capable of generating respective medical images depicting the vessel structure, which is a vessel structure of a patient, for example a human or animal, for example after a contrast agent has been applied. In particular, the imaging device may be an X-ray imaging device, in particular an X-ray-based angiography device, for example a C-arm device. The medical images may therefore be two-dimensional X-ray projection images. The medical images may, however, also be three-dimensional X-ray images, for example tomosynthesis reconstructions. The imaging device may also be a computed tomography, CT-, device, for example a CT-device for four-dimensional CT also denoted as time resolved CT, and the medical images may be three-dimensional CT-reconstructions. It is also possible that the imaging device is a magnetic resonance imaging, MRI-, device, in particular an MRI device for time resolved MRI, for example time resolved angiography.

In particular, each of the medical images depicts the vessel structure at a different time. In particular, the sequence of consecutive medical images corresponds to a sequence of consecutive frames and each graph representation corresponds to one of the frames. The sequence of consecutive medical images may consist of two or more medical images. Consequently, the sequence of consecutive frames may consist of two or more frames. Thus, the graph representations may consist of the first graph representation and the second graph representation or may comprise more than said two graph representations. In particular, there is a one-to-one correspondence between the graph representations and the sequence of medical images or the sequence of frames, respectively. However, the graph representations do not necessarily need to be processed in an ordered manner which is identical to the ordering of the medical images or frames, respectively.

It is also not necessary that the graph representations are received all at once, while this is possible in some implementations. In particular, all the graph representations may be received after the sequence of medical images has been generated. However, it is also possible that the graph representations are received one after another when the respective medical image has been generated.

It is noted that the augmented second graph representation may be augmented exactly once or multiple times depending on how many first segments of the first graph representation do not have respective counterparts in the second graph representation. It is also possible that the second graph representation comprises respective second segments for all first segments of the first graph representation from the beginning. In this case, no additional segment is added to the second graph representation. Nevertheless, the augmented second graph representation is also referred to in such cases.

The at least one aggregated representation may consist of the augmented second graph representation. In particular, the augmented second graph representation is an aggregated graph representation of the original first graph representation and second graph representation. It is, however, also possible that the augmented second graph representation is further processed to generate the at least one aggregated representation.

For example, the steps i) and ii) may be carried out in the same way as described also for a third graph representation and a fourth graph representation of the received graph representations, which differ from the first graph representation and the second graph representation. In this case, for example an augmented fourth graph representation is generated and the at least one aggregated representation is generated depending on the augmented second graph representation and the augmented fourth graph representation.

It is also possible that the steps i) and ii) are carried out again in the same way as described, wherein the augmented second graph representation takes the place of the first graph representation and a third graph representation of the received graph representations takes the place of the second graph representation. In this case, for example an augmented third graph representation is generated and the at least one aggregated representation is generated depending on the augmented second graph representation and the augmented third graph representation. This may, for example, be repeated analogously until all graph representations of the received graph representations are considered accordingly. It is also possible to group the graph representations into different groups and carry out the described procedures for each group individually.

It is, in particular, possible that, as a final result, a single aggregated representation is obtained. Said single aggregate representation would then have been generated depending on the augmented second graph representation.

The expression graph representation can, in particular, be understood as a graph in the context of graph theory. The nodes of a graph representation have respective attributes, namely at least the respective spatial coordinates, which may also be denoted as image coordinates. The spatial coordinates of the nodes of a given graph representation are, for example, given in a respective reference coordinate system of the corresponding graph or the corresponding medical image. Since the graph representation comprises the respective spatial coordinates for each node, the data processing system is able to determine whether for a given first segment the second graph representation comprises a corresponding second segment and, if this is not the case, augment the second graph representation as described. To check whether the second graph representation comprises a second segment corresponding to the respective first segment of the first graph representation, for example a coordinate-based distance metric between all nodes of the first segment and their closest counterparts in the second graph representation may, for example, be evaluated.

It is noted that the decision whether or not the second graph representation comprises a second segment corresponding to the respective first segment of the first graph representation can be based on different thresholds or tolerances depending on the actual embodiment of the computer-implemented method. This means that, in some implementations, it may be considered that the second graph representation does not comprise a second segment corresponding to the respective first segment, even though the second graph representation does comprise a part of such a second segment, which is, however, smaller or contains fewer nodes, respectively, than the respective first segment to an extent exceeding a predefined tolerance.

The extraction of the vessel structure from the respective medical image and the generation of the graph representations based on the medical images are not necessarily steps, which are comprised by the computer-implemented method according to the invention, and can be carried out using known techniques. However, in some embodiments, the steps of extracting the vessel structure from the respective medical image and generating the graph representations based on the medical images are part of the computer-implemented method.

In several embodiments, each of the graph representations is given by a graph representation 11, for example a rooted graph representation 11.

In particular, each node of one of the graph representations has either one parent node or no parent node. In each graph representation, exactly one node without a parent node exists and this node is denoted as root node. A node can have exactly one child node or more than one child nodes or no child node at all. Nodes, which have no child nodes are denoted as leaf nodes and nodes with more than one child node are denoted as multifurcation node, in particular bifurcation nodes for exactly two child nodes, trifurcation nodes for exactly three child nodes, and so forth.

Each graph representation comprises one or more segments, which may also be denoted as branches. Each segment comprises a respective subset of the nodes of the respective graph representation. In particular, a segment may be understood such that all nodes of the respective segment except an initial node of the segment have a parent node in the same segment and all nodes of the respective segment except a final node of the segment have exactly one child node in the same segment. Consequently, the initial node is either the root node or its parent node is part of another segment of the same graph representation. Analogously, the final node is either a leaf node or it has two or more child nodes in other segments of the same graph representation. In other words, a segment may, for example, be understood as a subset of pairwise connected nodes of a graph representation without multifurcations.

When assessing a vessel structure, for example in order to determine certain hemodynamic characteristics, in particular by a trained machine learning model, MLM, different phenomena may cause the individual medical images to not all show the same portions of the vessel structure and/or to not all show the same portions of the vessel structure completely. These phenomena may, for example, include body movement of the patient, cardiac or respiratory motion, the dynamics of the contrast agent bolus and so forth. These phenomena may further be accentuated in case the imaging settings or protocol for generating the medical images are not chosen in an optimal way, in particular in case of two-dimensional imaging. The respective algorithm for extracting the vessel structure and/or generating the graph representations based on the medical images may therefore not generate the same segments for all graph representations, even though they all represent the same vessel structure.

The computer-implemented method uses a graph aggregation approach achieving a robust aggregation of potentially inconsistent graph representations of the vessel structure, which were individually extracted from multiple frames of the same angiography sequence, in particular the same underlying anatomy and the same patient but at different time points, for example at different heart phases, different image quality and hence different quality of extracted graph representations. The at least one aggregated representation overcomes these inconsistencies at least partially. Using the at least one aggregated representation as a basis or as auxiliary information for downstream tasks, in particular for characterizing the hemodynamics in the vessel structure, improves the quality of the results of said downstream tasks and, consequently the quality of clinical decisions made based on said results and/or the chances of success of a medical intervention, which uses the results of said downstream tasks.

According to several implementations of a first embodiment, the graph representations, in particular all received graph representations, are ordered into a single ordered sequence of graph representations. The total number of graph representations of the ordered sequence is N. The graph representations may be indexed by integer numbers i from 1 to N, in other words $i \in$

$$[1, N] \cap \mathbb{N}$$ . N-1 iterations are carried out, wherein the N-1 iterations start with i=1 and end with i=N-1 and after each iteration, i is incremented by 1 until i=N-1. For each of the N-1 iterations and for each segment of the i-th graph representation of the ordered sequence,

> i) it is determined whether the (i+1)-th graph representation of the ordered sequence comprises a segment corresponding to the respective segment of the i-th graph representation of the ordered sequence, and
>
> ii) if it is found that the (i+1)-th graph representation does not comprise a segment corresponding to the respective segment of the i-th graph representation, the (i+1)-th graph representation is augmented by adding an additional segment corresponding to the respective segment of the i-th graph representation to the (i+1)-th graph representation.

The at least one aggregated representation is generated depending on, for example comprising or consisting of, the augmented N-th graph representation of the ordered sequence, in particular when all of the N-1 iterations have been carried out.

In other words, the steps explained above for the first graph representation and the second graph representation are carried out iteratively for each pair of consecutive graph representations in the ordered sequence. More specifically, in a given iteration i, the i-th graph representation corresponds to the first graph representation in the earlier explanations above and the (i+1)-th graph representation corresponds to the second graph representation in the earlier explanations above. It is noted that the first graph representation and the second graph representation are also part of the ordered sequence of graph representations. Consequently, the N-1 iterations as described also comprise one iteration, where the i-th graph representation is the first graph representation and the (i+1)-th graph representation is the second graph representation. The described steps are, in particular, not carried out twice for the identical first and second graph representation.

It is further noted that the ordering of the ordered sequence of graph representations may match the order ring of the sequence of consecutive medical images, but this is not necessarily the case.

In such embodiments, the augmented N-th graph representation should comprise all segments of all graph representations of the ordered sequence prior to the N-1 iterations. In particular, the augmented N-th graph representation is an aggregated graph representation of all graph representations of the ordered sequence. Therefore, the N-th graph representation is particularly suitable as an aggregated graph representation for the purposes explained above.

The at least one aggregated representation may consist of the augmented N-th graph representation. It is, however, also possible that the augmented N-th graph representation is further processed to generate the at least one aggregated representation.

According to several implementations of the first embodiment, after the N-1 iterations have been carried out, N-1 further iterations are carried out. For the further iterations, the graph representations may be indexed by integer numbers j from 1 to N, in other words $j \in [1, N] \cap \mathbb{N}$ . The N-1 further iterations start with j=1 and end with j=N-1 and after each iteration, j is incremented by 1 until j=N-1. For each iteration of the N-1 further iterations and for each segment of the (N-j+1)-th graph representation of the ordered sequence,

> iii) it is determined whether the (N-j)-th graph representation of the ordered sequence comprises a segment corresponding to the respective segment of the (N-j+1)-th graph representation of the ordered sequence;
>
> iv) if it is found that the (N-j)-th graph representation does not comprise a segment corresponding to the respective segment of the (N-j+1)-th graph representation, the (N-j)-th graph representation is augmented by adding an additional segment corresponding to the respective segment of the (N-j+1)-th graph representation to the (N-j)-th graph representation The at least one aggregated representation is generated depending on, for example comprising or consisting of, the augmented initial graph representation, which is the graph representation with j=1, of the ordered sequence, in particular when all of the N-1 further iterations have been carried out. For example, the at least one aggregated representation may be generated depending on, for example comprising or consisting of, all graph representations of the ordered sequence, after the N-1 further iterations have been carried out.

In other words, after the N-1 iterations have been carried out as described above, the N-1 further iterations are carried out based on the resulting ordered sequence in the opposite order. While the N-1 iterations start with the 1-th and the 2-th graph representation (i=1) and end with the (N-1)-th and N-th graph representation (i=N-1), the N-1 further iterations start with the N-th and the (N-1)-th graph representation (j=1) and end with the 2-th and 1-th graph representation (j=N-1).

In such embodiments, after the N-1 further iterations have been carried out, all graph representations of the ordered sequence should comprise all segments of all graph representations of the ordered sequence prior to the N-1 iterations. Therefore, after the N-1 further iterations have been carried out, the graph representations are particularly suitable as aggregated graph representations for the purposes explained above, in particular for downstream applications, where a graph representation is required for each frame of the sequence of consecutive frames.

According to several implementations of a second embodiment, the graph representations, in particular all received graph representations, are grouped into at least two ordered sequences of graph representations including a first ordered sequence and a second ordered sequence. The total number of graph representations of the first ordered sequence is N1. The graph representations of the first ordered sequence may be indexed by integer numbers i from 1 to N, in other words

$i \in [1, N1] \cap \mathbb{N}$. N1-1 iterations are carried out, wherein the N1-1 iterations start with i=1 and end with i=N1-1 and after each iteration, i is incremented by 1 until i=N1-1. For each iteration of the N1-1 iterations and for each segment of the i-th graph representation of the first ordered sequence,

  i) it is determined whether the (i+1)-th graph representation of the first ordered sequence comprises a segment corresponding to the respective segment of the i-th graph representation of the first ordered sequence, and

  ii) if it is found that the (i+1)-th graph representation of the first ordered sequence does not comprise a segment corresponding to the respective segment of the i-th graph representation of the first ordered sequence, the (i+1)-th graph representation of the first ordered sequence is augmented by adding an additional segment corresponding to the respective segment of the i-th graph representation of the first ordered sequence to the (i+1)-th graph representation of the first ordered sequence.

The at least one aggregated representation is generated depending on the augmented N1-th graph representation of the first ordered sequence, in particular when all of the N1-1 iterations have been carried out.

The explanations regarding the N-1 iterations of steps i) and ii) of the first embodiment hold analogously here for the second embodiment. In the implementations according to the second embodiment, however, the steps are not carried out for all of the graph representations one after the other, but only for a subset, namely the first ordered sequence. The other ordered sequences of the at least two ordered sequences, in particular the second ordered sequence, may be treated analogously. An advantage of such implementations is that the iterations may be carried out for the at least two ordered sequences in parallel, which reduces the overall required for the computation.

The at least two ordered sequences may be defined in an arbitrary way. For example, if the at least two ordered sequences consist of K ordered sequences, they may be defined such that each ordered sequences comprises 1/K or approximately 1/K of all the graph representations. According to several implementations of the second embodiment, the total number of graph representations of the second ordered sequence is N2. The graph representations of the second ordered sequence may be indexed by integer numbers i from 1 to N, in other words $i \in$ $[1, N2] \cap \mathbb{N}$. N2-1 further iterations are carried out, wherein the N2-1 further iterations start with i=1 and end with i=N1-1 and after each further iteration, i is incremented by 1 until i=N2-1. For each further iteration of the N2-1 further iterations and for each segment of the i-th graph representation of the second ordered sequence,

  iii) it is determined whether the (i+1)-th graph representation of the second ordered sequence comprises a segment corresponding to the respective segment of the i-th graph representation of the second ordered sequence, and

  iv) if it is found that the (i+1)-th graph representation of the second ordered sequence does not comprise a segment corresponding to the respective segment of the i-th graph representation of the second ordered sequence, the (i+1)-th graph representation of the second ordered sequence is augmented by adding an additional segment corresponding to the respective segment of the i-th graph representation of the second ordered sequence to the (i+1)-th graph representation of the second ordered sequence.

The at least one aggregated representation is generated depending on the augmented N2-th graph representation of the second ordered sequence, in particular when all of the N2-1 iterations have been carried out.

According to several implementations of the second embodiment, the N1-1 iterations, in particular steps i) and ii), and the N2-1 further iterations, in particular steps iii) and iv), are carried out in parallel or partially in parallel.

For example, different data processing devices or computing cores or the like may be used to carry out the N1-1 iterations and the N2-1 iterations, respectively.

According to several implementations of the second embodiment, for each segment of the N1-th graph representation of the first ordered sequence,

v) it is determined whether the N2-th graph representation of the second ordered sequence comprises a segment corresponding to the respective segment of the N1-th graph representation of the first ordered sequence, and

vi) if it is found that the N2-th graph representation of the second ordered sequence does not comprise a segment corresponding to the respective segment of the N1-th graph representation of the first ordered sequence, the N2-th graph representation of the second ordered sequence is augmented by adding an additional segment corresponding to the respective segment of the N1-th graph representation of the first ordered sequence to the N2-th graph of the second ordered sequence.

The at least one aggregated representation is generated depending on the augmented N2-th graph representation of the second ordered sequence.

In such embodiments, the augmented N2-th graph representation should comprise all segments of all graph representations of the first ordered sequence prior to the N1-1 iterations and the second ordered sequence prior to the N2-1 further iterations. Therefore, the N2-th graph representation is particularly suitable as an aggregated graph representation for the purposes explained above.

As explained for the respective implementations of the first embodiment, also in implementations of the second embodiment, additional aggregation steps may be carried out in the reverse order.

According to several implementations of the computer-implemented method, the first graph representation is registered to the second graph representation, in particular depending on the spatial coordinates of the nodes of the first graph representation and the spatial coordinates of the nodes of the second graph representation. A predefined distance metric between the registered respective segment of the first graph representation and the second graph representation is computed, in particular depending on the spatial coordinates of the nodes of the respective segment of the first graph representation after the registration and the spatial coordinates of the nodes of the second graph representation. It is determined whether the second graph representation comprises a segment corresponding to the respective segment of the first graph representation depending on the computed distance metric.

For example, if the computed distance metric is greater than a predefined threshold value, then the segment may be considered missing in the second graph representation. The registration can be a rigid registration or a deformable registration. In particular, registering the first graph representation to the second graph representation comprises finding a transformation, which maps the nodes of the first graph representation to corresponding nodes of the second graph representation, in case the second graph representation comprises such corresponding nodes. The registered segment of the first graph representation is then given by the accordingly transformed segment of the first graph representation.

By means of the registration, it is explicitly accounted for a possible movement of the patient or the vessel structure while the medical images have been acquired. Thus, the accuracy of the aggregation is increased.

Instead of said registration approach, it is also possible to apply an accordingly trained MLM to the first graph representation and the second graph representation to identify all segments of the first graph representation which do not have a corresponding counterpart in the second graph representation.

According to several implementations of the computer-implemented method, a deformation field between the first graph representation and the second graph representation is determined, in particular depending on the spatial coordinates of the nodes of the first graph representation and the spatial coordinates of the nodes of the second graph representation. The second graph representation is augmented by adding the additional segment to the second graph representation depending on the deformation field.

The deformation field is, in particular, a transformation which maps each node of the first graph representation into the coordinate system of the second graph representation, such that the resulting transformed first graph representation optimally matches the second graph representation. Finding the deformation field can be considered and solved as an optimization problem using known methods, in particular methods known from deformable image registration techniques.

In principle, it is also possible to augment the second graph representation by adding the additional segment without the deformation field, in particular without any such transformation. This implicitly or explicitly assumes that the graph representations corresponding to different frames have not significantly moved with respect to each other. By adapting the coordinates of the nodes of the additional segment according to the deformation field, the accuracy of the proposed method is therefore increased.

According to a further aspect of the invention, a method for generating at least one aggregated representation of a vessel structure, which is not purely computer-implemented, is provided. Therein, a sequence of consecutive medical images depicting the vessel structure is generated by an imaging device and a respective graph representation of the vessel structure is generated for each of the sequence of consecutive medical images, for example by the data processing system. A computer-implemented method according to the invention is carried out based on the graph representations, in particular by the data processing system.

According to several implementations of the method, the sequence of consecutive medical images is generated as a sequence of consecutive two-dimensional images, for example two-dimensional X-ray images.

In such implementations, the invention is particularly beneficial since, due to the two-dimensional nature of the medical images, the chances that parts of the vessel structure are not consistently depicted in each medical image, for example due to occlusion by other parts of the vessel structure or by other objects, are increased. Consequently, the at least one aggregated representation of the vessel structure is particularly helpful in this case.

According to several implementations, the sequence of consecutive medical images contains medical images from at least two viewing directions to the vessel structure.

Also in such implementations, the invention is particularly beneficial, since it often cannot be guaranteed that the optimal imaging settings for both viewing directions are chosen by the operator, such that the chances for inconsistency among the medical images is also increased.

According to a further aspect of the invention, a method for analyzing a hemodynamics characteristics of a vessel structure is provided. Therein, a method or computer-implemented method for generating at least one aggregated representation of the vessel structure according to the invention is carried out. A predefined algorithm for analyzing the hemodynamics characteristics of the vessel structure is carried out depending on the at least one aggregated representation.

For example, the analysis of the hemodynamics characteristics may comprise determining a fractional flow reserve, FFR, of the vessel structure, carrying out a vessel detection of the vessel structure, carrying out a segmentation, for example a branch segmentation, of the vessel structure, carrying out a branch labeling of the vessel structure, and so forth.

According to several implementations, the algorithm for analyzing the hemodynamics characteristics comprises a trained machine learning model, MLM, which has been trained to analyze the hemodynamics characteristics using a known training technique.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network, GAN.

According to a further aspect of the invention, a data processing system is provided. The data processing system is configured to carry out a computer-implemented method according to the invention.

In the present disclosure, the expressions "data processing system" and "at least one data processing device" may be used interchangeably. A data processing device may in particular be understood as a data processing device which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a medical imaging system is provided. The medical imaging system comprises an imaging device, which is configured to generate a sequence of consecutive medical images depicting a vessel structure, for example an X-ray imaging device, in particular an X-ray angiography device and/or a C-arm device. The medical imaging system comprises a data processing system, which is configured to generate a respective graph representation of the vessel structure for each medical image of the sequence of consecutive medical images, and to carry out a computer-implemented method for generating at least one aggregated representation of the vessel structure according to the invention based on the graph representations.

Further implementations of the medical imaging system according to the invention follow directly from the various embodiments of the method and the computer-implemented method according to the invention and vice versa. In particular, individual features and corresponding explanations as well as advantages relating to the various implementations of the method and the computer-implemented method according to the invention can be transferred analogously to corresponding implementations of the medical imaging system according to the invention. In particular, the medical imaging system according to the invention is designed or programmed to carry out the method or the computer-implemented method according to the invention. In particular, the medical imaging system according to the invention carries out the method or the computer-implemented method according to the invention.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can, for example, be provided as binary code or assembler and/or as source code of a programming language, for example C, and/or as program script, for example Python.

According to a further aspect of the invention, a further computer program comprising further instructions is provided. When the further instructions are executed by a medical imaging system according to the invention, in particular by the data processing system of the medical imaging system, the instructions cause the medical imaging system to carry out a method for generating at least one aggregated representation of a vessel structure according to the invention or a method for analyzing a hemodynamics characteristics of a vessel structure according to the invention.

The further instructions may be provided as program code, for example. The program code can for example be provided as binary code or assembler and/or as source code of a programming language, for example C, and/or as program script, for example Python.

According to a further aspect of the invention, a computer-readable storage medium storing a computer program and/or a further computer program according to the invention is provided.

The computer program, the further computer program, and the computer-readable storage medium are respective computer program products comprising the instructions and/or the further instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,

FIG 1   shows schematically an exemplary implementation of a medical imaging system according to the invention;

FIG 2   shows schematically a medical image depicting a vessel structure and a corresponding graph representation of the vessel structure;

FIG 3   shows a schematic flow diagram of an exemplary implementation of a computer-implemented method for generating at least one aggregated representation of a vessel structure according to the invention;

FIG 4   shows a schematic flow diagram of a further exemplary implementation of a computer-implemented method for generating at least one aggregated representation of a vessel structure according to the invention;

FIG 5   shows a schematic flow diagram of a further exemplary implementation of a computer-implemented method for generating at least one aggregated representation of a vessel structure according to the invention; and

FIG 6   shows schematically an artificial neural network.

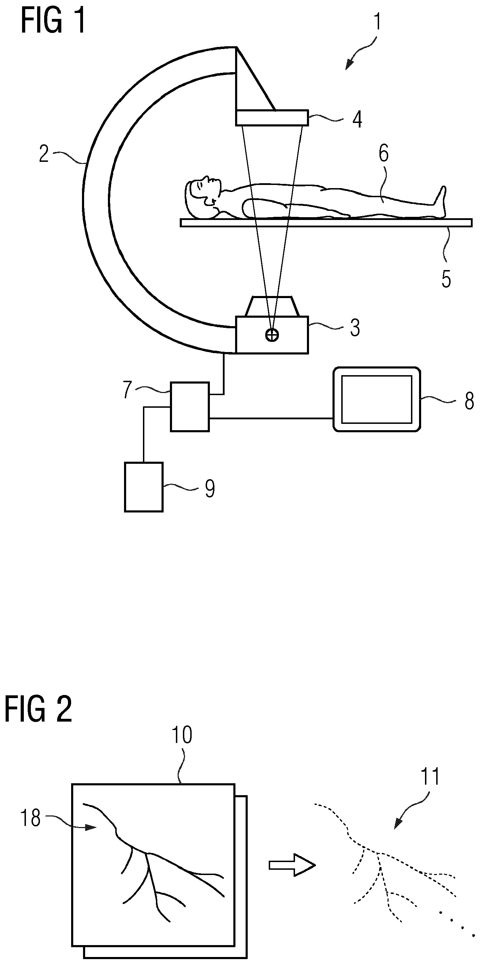

FIG 1 shows schematically an exemplary implementation of a medical imaging system 1 according to the invention. The medical imaging system comprises an imaging device, for example an X-ray imaging device.

The X-ray imaging device comprises a source unit 3 with an X-ray source, a detector unit 4 with an X-ray detector, and a control system 7, which is configured to control the X-ray source and the X-ray detector to generate X-ray images 10 depicting a vessel structure 18, for example a coronary vessel structure, of a patient 6, as depicted schematically in FIG 2. The X-ray imaging device may, for example, comprise a patient table 5, on which the patient 6 is arranged.

The medical imaging system 1 comprises a data processing system 9 according to the invention, which is adapted to carry out a computer-implemented method for generating at least one aggregated representation of the vessel structure 18 according to the invention. In the following, several functions and method steps are described to be carried out by the control system 7, while other functions and method steps are described to be carried out by the data processing system 9. It is noted that the functions and method steps may also be distributed in different ways in alternative implementations.

For example, the control system 7 may adjust various imaging parameters of the X-ray imaging device including, for example, exposure parameters like a peak kilovoltage of the X-ray source, a tube current of the X-ray source, and/or an X-ray pulse duration. For example, the control system 7 may adjust further imaging parameters like a filter material and/or filter thickness of an X-ray filter, for example a copper filter, by placing the appropriate X-ray filter into the beam path or by removing it from the beam path, respectively. For example, the control system 7 may adjust further imaging parameters like a collimator opening size of an X-ray collimator. For example, the control system 7 may bring the X-ray collimator into the beam path or remove it from the beam path, respectively. For example, the control system 7 may adjust further imaging parameters like a gain factor of the X-ray detector. For example, the control system 7 may bring an anti-scattering grid into the beam path or remove it from the beam path, respectively.

In some implementations, the X-ray imaging device comprises a display device 8, wherein the control system 7 is configured to control the display device 8 to display the X-ray images 10 or processed X-ray images.

In some implementations, the X-ray imaging device is designed as a C-arm fluoroscopy system, wherein the source unit 3 and the detector unit 4 are attached opposite to each other on a C-arm 2, which can be rotated around different axes. The corresponding motions are denoted as angular and orbital motion respectively. In some implementations, apart from the rotational motion of the C-arm 2, the patient table 5 and the C-arm 2 may be positioned relative to each other by respective translatory motions of the C-arm 2 and/or the patient table 5. Consequently, the position and/or orientation of the X-ray source with respect to the object 6 and the position and/or orientation of the X-ray detector with respect to the object 6 may be adjusted exactly to the required imaging perspective.

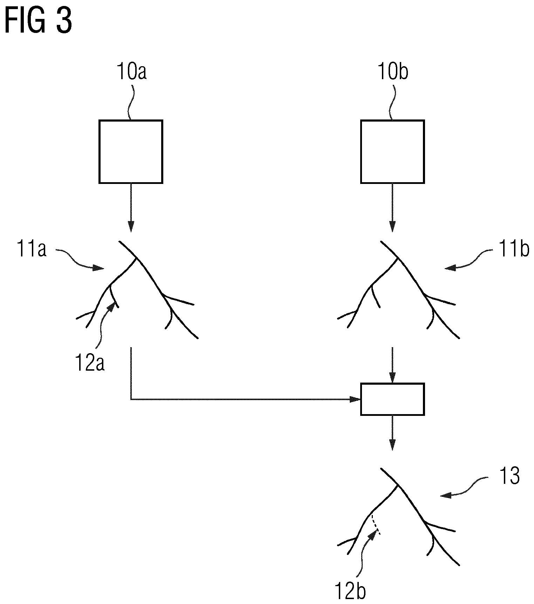

FIG 3 shows a schematic flow diagram of an exemplary implementation of a computer-implemented method for generating at least one aggregated representation of a vessel structure 18 according to the invention. Therein, the data processing system 9 receives a sequence of consecutive medical images 10, 10a, 10b depicting the vessel structure 18 from the medical imaging device and generates graph representations 11, 11a, 11b, in particular rooted graph representations 11, of the vessel structure 18, each of the graph representations 11, 11a, 11b corresponding to one of the medical images 10, 10a, 10b of. Each of the graph representations 11, 11a, 11b comprises a plurality of nodes and respective spatial coordinates for each node, wherein the plurality of nodes is arranged according to at least one segment 12a of the respective graph representation 11, 11a, 11b.

For each segment 12a of a first graph representation 11a of the plurality of graph representations 11, 11a, 11b, it is determined whether a second graph representation 11b of the plurality of graph representations 11, 11a, 11b comprises a segment corresponding to the respective segment 12a of the first graph representation 11, 11a, 11b. If it is found that the second graph representation 11b does not comprise a segment corresponding to the respective segment 12a of the first graph representation 11a, the second graph representation 11b is augmented by adding an additional segment 12b corresponding to the respective segment 12a of the first graph representation 11a to the second graph representation 11b. The at least one aggregated representation is generated depending on the augmented second graph representation 13.

In the example of FIG 3, the first graph representation 11a comprises exactly one segment 12a, which does not have a respective counterpart in the second graph representation 11b. Thus, the augmented second graph representation 13 corresponds to the second graph representation 11b with the additional segment 12b. In general, however, it is also possible that there is no such additional segment 12b to be added or there is more than one additional segment 12b to be added.

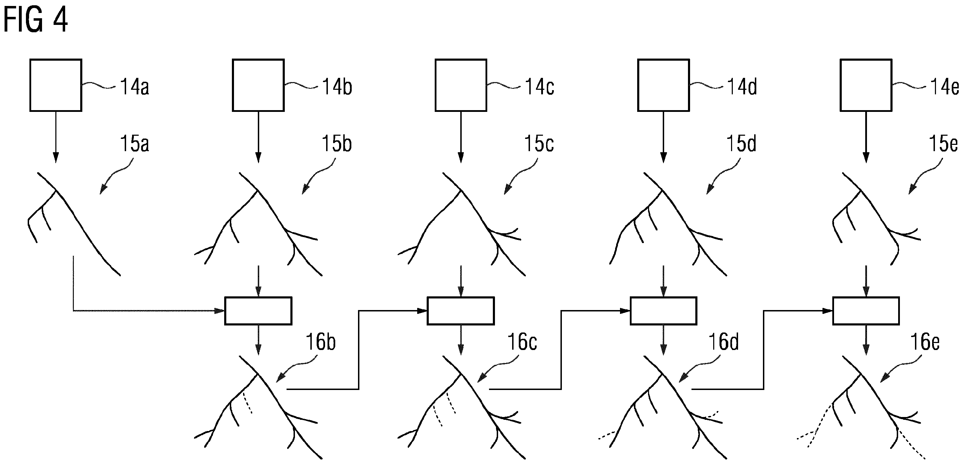

The total number of the medical images 10 may be greater than two and, consequently, the number of graph representations 11 to be aggregated may be greater than two as well. As an example, FIG 4 depicts schematically a scenario with five medical images 14a, 14b, 14c, 14d, 14e and five corresponding graph representations 15a, 15b, 15c, 15d, 15e extracted from the medical images 14a, 14b, 14c, 14d, 14e.

In a further exemplary implementation of the computer-implemented method according to the invention, the aggregation is carried out as described in the following. First, the graph representation 15b is compared to the preceding graph representation 15a. An augmented graph representation 16b is generated by adding respective segments of the graph representation 15a, which do not have a corresponding counterpart in the graph representation 15b, to the graph representation 15b. Then, the graph representation 15c is compared to the augmented graph representation 16b. An augmented graph representation 16c is generated by adding respective segments of the augmented graph representation 16b, which do not have a corresponding counterpart in the graph representation 15c, to the graph representation 15c.

Then, the graph representation 15d is compared to the augmented graph representation 16c. An augmented graph representation 16d is generated by adding respective segments of the augmented graph representation 16c, which do not have a corresponding counterpart in the graph representation 15d, to the graph representation 15d. Finally, the graph representation 15e is compared to the augmented graph representation 16d. An augmented graph representation 16e is generated by adding respective segments of the augmented graph representation 16d, which do not have a corresponding counterpart in the graph representation 15e, to the graph representation 15e. Consequently, the resulting augmented graph representation 16e comprises all segments of all of the graph representations 15a, 15b, 15c, 15d, 15e.

The augmented graph representation 16e may be a result of the computer-implemented method, for example in a scenario where the aggregation is carried out online or live, right after the respective medical image 14a, 14b, 14c, 14d, 14e has been generated. Once another medical image is generated, the next aggregation step may be carried out analogously.

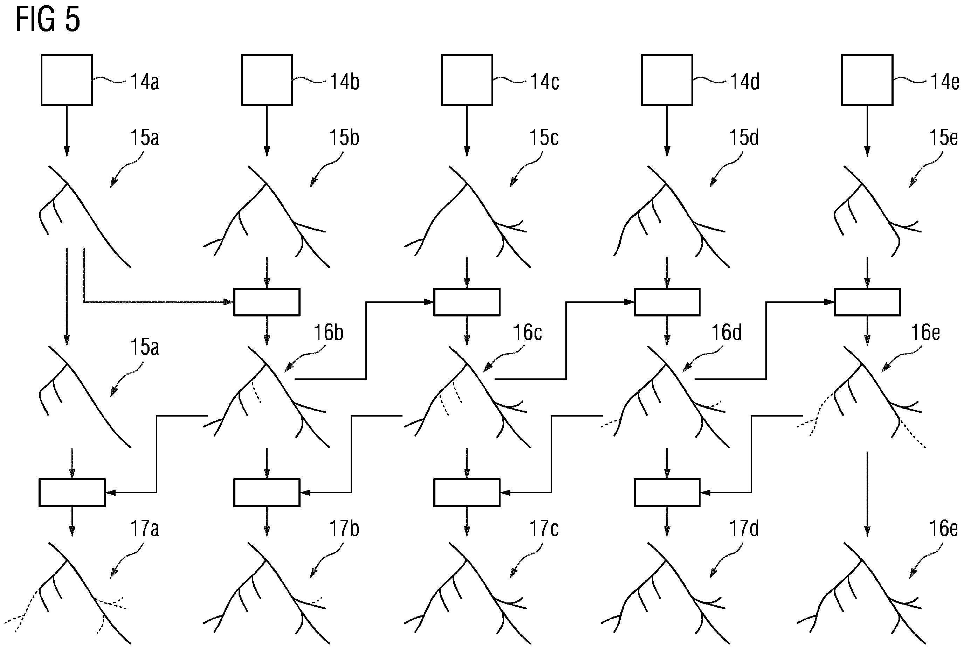

Alternatively, the medical images 14a, 14b, 14c, 14d, 14e may have been generated previously and afterwards the aggregation is carried out. A corresponding further exemplary implementation of the computer-implemented method according to the invention is shown schematically in FIG 5.

The steps as explained with respect to FIG 4 are carried out also in this implementation, they may be denoted as forward processing. Afterwards, the augmented graph representations 16b, 16c, 16d, 16e are processed in the opposite order, which may be denoted as backwards processing.

In particular, the augmented graph representation 16d is compared to the augmented graph representation 16e. An augmented graph representation 17d is generated by adding respective segments of the augmented graph representation 16e, which do not have a corresponding counterpart in the augmented graph representation 16d, to the augmented graph representation 16d. Then, the augmented graph representation 16c is compared to the augmented graph representation 16d. An augmented graph representation 17c is generated by adding respective segments of the augmented graph representation 16d, which do not have a corresponding counterpart in the augmented graph representation 16c, to the augmented graph representation 16c. Then, the augmented graph representation 16b is compared to the augmented graph representation 16c. An augmented graph representation 17b is generated by adding respective segments of the augmented graph representation 16c, which do not have a corresponding counterpart in the augmented graph representation 16b, to the augmented graph representation 16b. Finally, the graph representation 15a is compared to the augmented graph representation 16b. An augmented graph representation 17a is generated by adding respective segments of the augmented graph representation 16b, which do not have a corresponding counterpart in the graph representation 15a, to the graph representation 15a.

The resulting augmented graph representations 17a, 17b, 17c, 17d, 16e each comprise all segments of all of the graph representations 15a, 15b, 15c, 15d, 15e.

In some implementations, during the forward and backward processing, a method of mapping the locations of all nodes pertaining to a graph representation 15a, 15b, 15c, 15d, 15e in a given frame onto corresponding locations in another frame is employed, which will provide more accurate results. This can be accomplished, for example, by applying pre-computed deformation fields that take into account the temporal movement of the vessel structure 18, for example due to cardiac motion, respiratory motion, table panning etc. over time. These deformation fields can be computed based on the angiographic data and/or based on additional information previously extracted, for example vesselness heatmaps, either via a standard deformable registration algorithm, or by applying an MLM, for example a deep neural network, trained specifically for this task. Thus, merging segments pertaining to graph representation 15a, 15b, 15c, 15d, 15e extracted from two different frames employs these deformation fields to bring the node locations in the same coordinate space, then a fuzzy check may be used to determine whether that segment or some portion of it is already covered or not, for example by means of calculating the distances of the segment's nodes to the closest respective node and applying thresholding or similar techniques.

In other implementations, instead of performing the aggregation sequentially across frames as described with regard to FIG 4 and FIG 5, a divide-and-conquer approach may be employed. Such approaches process the graph representations 15a, 15b, 15c, 15d, 15e in a similar fashion to a merge-sort algorithm, by dividing the sequence of graph representations 15a, 15b, 15c, 15d, 15e into subsequences until the trivial subsequence containing two graph representations 15a, 15b, 15c, 15d, 15e is reached, then merging the graph representations 15a, 15b, 15c, 15d, 15e in a bottom-up fashion.

In some implementations, a post-processing step ensuring segment ordering consistency may be performed by adjusting the order of child nodes for the multifurcation nodes such that a tree traversal algorithm, for example a depth first traversal algorithm, applied on any two trees yields pairs of corresponding segments.

In some implementations, segments for which there is not enough supporting evidence, for example a segment being detected only in a small subset of frames, or a segment having a different trace in a subset of frames due to overlaps, may be removed from the aggregated tree model or marked as uncertain.

In several implementations of the present invention, a task is to aggregate graph representations 11 of a vessel structure 18, extracted from multiple frames into a unified aggregated graph representation, which is designed to robustly aggregate topological information of the vessel structure into a frame-agnostic topology model. This allows establishing correspondences to match specific portions of the vessel structure 18, for example side branches, landmarks, bifurcation points, and so forth, across frames. Accurate matching at the topology level is very helpful for subsequent analysis tasks involving multiple frames.

In several implementations of the present invention, a tree aggregation method is performed, which is tailored towards robust aggregation of noisy coronary topology graph representations 11, which were individually extracted from multiple frames of the same coronary angiography sequence, that is the same underlying anatomy and the same patient 6, but at different time points and/or different heart phases and/or different image quality and hence different quality of the extracted graph representations 11. The graph representations 11 may also implicitly hold an order of branches or segments or more generally, subsets of nodes in the graph representations 11, which can be used to uniquely identify a subset of nodes in the graph representation 11, for example using a numeric identifier. This ordering may represent, for example, the hemodynamic significance of each segment as determined based on geometric attributes such as length and radius.

It is noted, however, that the method for extracting the graph representations 11 from the medical images 10 is not necessarily required to generate a consistent ordering across multiple frames. Furthermore, there may be inconsistencies, for example in the number of extracted segments and in their ordering.

For example, a trained MLM, for example a trained artificial neural network, ANN, may be applied to the at least one aggregated representation to analyze a hemodynamics characteristics of the vessel structure 18, for example to compute an FFR.

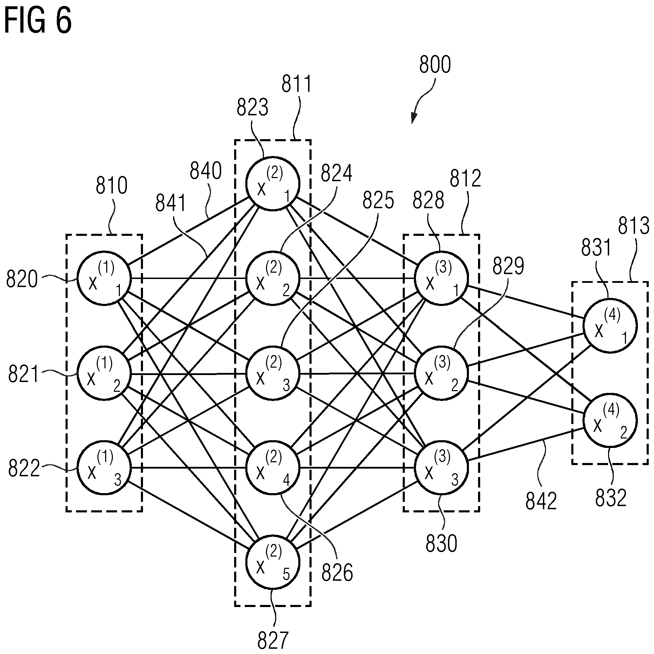

FIG 6 displays an embodiment of an ANN 800, which is for example designed as a multi-layer perceptron, MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 6, the edge 840 is a directed connection from the node 820 to the node 823, and the edge 842 is a directed connection from the node 830 to the node 832. An edge 840, ..., 842 from a first node 820, ..., 832 to a second node 820, ...,

832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, $x^{(n)}_i$ denotes the value of the i-th node 820, ..., 832 of the n-th layer 810, ..., 813. The values of the nodes 820, ..., 822 of the input layer 810 are equivalent to the input values of the artificial neural network 800. The values of the nodes 831, 832 of the output layer 813 are equivalent to the output value of the artificial neural network 800.

Furthermore, each edge 840, ... , 842 can comprise a weight being a real number. In particular, the weight is a real number within the interval [-1, 1] or within the interval [0, 1]. Here, $w^{(m,n)}_{i,j}$ denotes the weight of the edge between the i-th node 820, ... , 832 of the m-th layer 810, ... , 813 and the j-th node 820, ... , 832 of the n-th layer 810, ... , 813. Furthermore, the abbreviation $w^{(n)}_{i,j}$ is defined for the weight $w^{(n,n+1)}_{i,j}$. In particular, to calculate the output values of the neural network 800, the input values are propagated through the neural network 800. In particular, the values of the nodes 820, ... , 832 of the (n+1)-th layer 810, ... , 813 can be calculated based on the values of the nodes 820, ... , 832 of the n-th layer 810, ..., 813 by

$$x_j^{(n+1)} = f\left(\sum_i x_i^{(n)}\, w_{i,j}^{(n)}\right).$$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is, for example, used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to

$$w'^{(n)}_{i,j} = w^{(n)}_{i,j} - \gamma \, \delta^{(n)}_j \, x^{(n)}_i,$$

wherein $\gamma$ is a predefined learning rate, and the numbers $\delta^{(n)}_j$ can be recursively calculated as

$$\delta^{(n)}_j = \left( \sum_k \delta^{(n+1)}_k \, w^{(n+1)}_{j,k} \right) f' \left( x^{(n)}_i w^{(n)}_{i,j} \right)$$

based on $\delta^{(n+1)}_j$, if the (n+1)-th layer is not the output layer 813, and

$$\delta^{(n)}_j = \left( x^{(n+1)}_j - t^{(n+1)}_j \right) \, f' \left( x^{(n)}_i w^{(n)}_{i,j} \right),$$

if the (n+1)-th layer is the output layer 813, wherein f' is the first derivative of the activation function, and $t^{(n+1)}_j$ is the comparison training value for the j-th node of the output layer 813.

Independent of the grammatical term usage, individuals with male, female or other gender identities are included within the term.

**Claims**

1. Computer-implemented method for generating at least one aggregated representation of a vessel structure (18), wherein

   - graph representations (11, 11a, 11b, 13, 15a-15e, 16b-16e, 17a-17d) of the vessel structure (18) are received, each of the graph representations (11, 11a, 11b, 13, 15a-15e, 16b-16e, 17a-17d) corresponding to a respective medical image (10, 10a, 10b, 14a-14e) of a sequence of consecutive medical images (10, 10a, 10b, 14a-14e) depicting the vessel structure (18);
   - each of the graph representations (11, 11a, 11b, 13, 15a-15e, 16b-16e, 17a-17d) comprises a plurality of nodes and respective spatial coordinates for each node, wherein the plurality of nodes is arranged according to at least one segment (12a, 12b) of the respective graph representation (11, 11a, 11b, 13, 15a-15e, 16b-16e, 17a-17d);
   - for each segment (12a, 12b) of a first graph representation (11, 11a, 11b, 13, 15a-15e, 16b-16e, 17a-17d) of the plurality of graph representations (11, 11a, 11b, 13, 15a-15e, 16b-16e, 17a-17d),

      i) it is determined whether a second graph representation (11, 11a, 11b, 13, 15a-15e, 16b-16e, 17a-17d) of the plurality of graph representations (11, 11a, 11b, 13, 15a-15e, 16b-16e, 17a-17d) comprises a segment (12a, 12b) corresponding to the respective segment (12a, 12b) of the first graph representation (11, 11a, 11b, 13, 15a-15e, 16b-16e, 17a-17d);
      ii) if it is found that the second graph representation (11, 11a, 11b, 13, 15a-15e, 16b-16e, 17a-17d) does not comprise a segment (12a, 12b) corresponding to the respective segment (12a, 12b) of the first graph representation (11, 11a, 11b, 13, 15a-15e, 16b-16e, 17a-17d), the second graph representation (11, 11a, 11b, 13, 15a-15e, 16b-16e, 17a-17d) is augmented by adding an additional segment (12a, 12b) corresponding to the respective segment (12a, 12b) of the first graph representation (11, 11a, 11b, 13, 15a-15e, 16b-16e, 17a-17d) to the second graph representation (11, 11a, 11b, 13, 15a-15e, 16b-16e, 17a-17d);

   - the at least one aggregated representation is generated depending on the augmented second graph representation (11, 11a, 11b, 13, 15a-15e, 16b-16e, 17a-17d).

2. Computer-implemented method according to claim 1, wherein

   - the graph representations (11, 11a, 11b, 13, 15a-15e, 16b-16e, 17a-17d) are ordered into a single ordered sequence of graph representations (11, 11a, 11b, 13, 15a-15e, 16b-16e, 17a-17d);
   - for each iteration of N-1 iterations, wherein the total number of graph representations (11, 11a, 11b, 13, 15a-15e, 16b-16e, 17a-17d) of the ordered sequence is N, the graph representations (11, 11a, 11b, 13, 15a-15e, 16b-16e, 17a-17d) are indexed by $i \in [1, N] \cap \mathbb{N}$, and the N-1 iterations start with i=1 and end with i=N-1, for each segment (12a, 12b) of the i-th graph representation (11, 11a, 11b, 13, 15a-15e, 16b-16e, 17a-17d) of the ordered sequence

    i) it is determined whether the (i+1)-th graph representation (11, 11a, 11b, 13, 15a-15e, 16b-16e, 17a-17d) of the ordered sequence comprises a segment (12a, 12b) corresponding to the respective segment (12a, 12b) of the i-th graph representation (11, 11a, 11b, 13, 15a-15e, 16b-16e, 17a-17d) of the ordered sequence;
    ii) if it is found that the (i+1)-th graph representation (11, 11a, 11b, 13, 15a-15e, 16b-16e, 17a-17d) does not comprise a segment (12a, 12b) corresponding to the respective segment (12a, 12b) of the i-th graph representation (11, 11a, 11b, 13, 15a-15e, 16b-16e, 17a-17d), the (i+1)-th graph representation (11, 11a, 11b, 13, 15a-15e, 16b-16e, 17a-17d) is augmented by adding an additional segment (12a, 12b) corresponding to the respective segment (12a, 12b) of the i-th graph representation (11, 11a, 11b, 13, 15a-15e, 16b-16e, 17a-17d) to the (i+1)-th graph representation (11, 11a, 11b, 13, 15a-15e, 16b-16e, 17a-17d);

    - the at least one aggregated representation is generated depending on the augmented N-th graph representation (11, 11a, 11b, 13, 15a-15e, 16b-16e, 17a-17d) of the ordered sequence.

3. Computer-implemented method according to claim 2, wherein, after the N-1 iterations have been carried out, for each iteration of N-1 further iterations, wherein the graph representations (11, 11a, 11b, 13, 15a-15e, 16b-16e, 17a-17d) are indexed by $j \in [1, N] \cap \mathbb{N}$, and the N-1 iterations start with j=1 and end with j=N-1, for each segment (12a, 12b) of the (N-j+1)-th graph representation (11, 11a, 11b, 13, 15a-15e, 16b-16e, 17a-17d) of the ordered sequence

    iii) it is determined whether the (N-j)-th graph representation (11, 11a, 11b, 13, 15a-15e, 16b-16e, 17a-17d) of the ordered sequence comprises a segment (12a, 12b) corresponding to the respective segment (12a, 12b) of the (N-j+1)-th graph representation (11, 11a, 11b, 13, 15a-15e, 16b-16e, 17a-17d) of the ordered sequence;
    iv) if it is found that the (N-j)-th graph representation (11, 11a, 11b, 13, 15a-15e, 16b-16e, 17a-17d) does not comprise a segment (12a, 12b) corresponding to the respective segment (12a, 12b) of the (N-j+1)-th graph representation (11, 11a, 11b, 13, 15a-15e, 16b-16e, 17a-17d), the (N-j)-th graph representation (11, 11a, 11b, 13, 15a-15e, 16b-16e, 17a-17d) is augmented by adding an additional segment (12a, 12b) corresponding to the respective segment (12a, 12b) of the (N-j+1)-th graph representation (11, 11a, 11b, 13, 15a-15e, 16b-16e, 17a-17d) to the (N-j)-th graph representation (11, 11a, 11b, 13, 15a-15e, 16b-16e, 17a-17d);

    - the at least one aggregated representation is generated depending on the augmented initial graph representation (11, 11a, 11b, 13, 15a-15e, 16b-16e, 17a-17d) of the ordered sequence.

4. Computer-implemented method according to claim 1, wherein

    - the graph representations (11, 11a, 11b, 13, 15a-15e, 16b-16e, 17a-17d) are grouped into at least two ordered sequences of graph representations (11, 11a, 11b, 13, 15a-15e, 16b-16e, 17a-17d) including a first ordered sequence and a second ordered sequence;
    - for each iteration of N1-1 iterations, wherein the total number of graph representations (11, 11a, 11b, 13, 15a-15e, 16b-16e, 17a-17d) of the first ordered sequence is N1, the graph representations (11, 11a, 11b, 13, 15a-15e, 16b-16e, 17a-17d) of the first ordered sequence are indexed by $i \in [1, N1] \cap \mathbb{N}$, and the N1-1 iterations start with i=1 and end with i=N1-1, for each segment (12a, 12b) of the i-th graph representation (11, 11a, 11b, 13, 15a-15e, 16b-16e, 17a-17d) of the first ordered sequence

    i) it is determined whether the (i+1)-th graph representation (11, 11a, 11b, 13, 15a-15e, 16b-16e, 17a-17d) of the first ordered sequence comprises a segment (12a, 12b) corresponding to the respective segment (12a, 12b) of the i-th graph representation (11, 11a, 11b, 13, 15a-15e, 16b-16e, 17a-17d) of the first ordered sequence;
    ii) if it is found that the (i+1)-th graph representation (11, 11a, 11b, 13, 15a-15e, 16b-16e, 17a-17d) of the first ordered sequence does not comprise a segment (12a, 12b) corresponding to the respective segment (12a, 12b) of the i-th graph representation (11, 11a, 11b, 13, 15a-15e, 16b-16e, 17a-17d) of the first ordered sequence, the (i+1)-th graph representation (11, 11a, 11b, 13, 15a-15e, 16b-16e, 17a-17d) of the first ordered sequence is augmented by adding an additional segment (12a, 12b) corresponding to the respective segment (12a, 12b) of the i-th graph representation (11, 11a, 11b, 13, 15a-15e, 16b-16e, 17a-17d) of the first ordered sequence to the (i+1)-th graph representation (11, 11a, 11b, 13, 15a-15e, 16b-16e, 17a-17d) of the first ordered sequence;

- the at least one aggregated representation is generated depending on the augmented N1-th graph representation (11, 11a, 11b, 13, 15a-15e, 16b-16e, 17a-17d) of the first ordered sequence.

5. Computer-implemented method according to claim 4, wherein

- for each iteration of N2-1 further iterations, wherein the total number of graph representations (11, 11a, 11b, 13, 15a-15e, 16b-16e, 17a-17d) of the second ordered sequence is N2, the graph representations (11, 11a, 11b, 13, 15a-15e, 16b-16e, 17a-17d) of the second ordered sequence are indexed by

$$i \in [1, N2] \cap \mathbb{N}$$, and the N2-1 further iterations start with i=1 and end with i=N2-1, for each segment (12a, 12b) of the i-th graph representation (11, 11a, 11b, 13, 15a-15e, 16b-16e, 17a-17d) of the second ordered sequence

iii) it is determined whether the (i+1)-th graph representation (11, 11a, 11b, 13, 15a-15e, 16b-16e, 17a-17d) of the second ordered sequence comprises a segment (12a, 12b) corresponding to the respective segment (12a, 12b) of the i-th graph representation (11, 11a, 11b, 13, 15a-15e, 16b-16e, 17a-17d) of the second ordered sequence;
iv) if it is found that the (i+1)-th graph representation (11, 11a, 11b, 13, 15a-15e, 16b-16e, 17a-17d) of the second ordered sequence does not comprise a segment (12a, 12b) corresponding to the respective segment (12a, 12b) of the i-th graph representation (11, 11a, 11b, 13, 15a-15e, 16b-16e, 17a-17d) of the second ordered sequence, the (i+1)-th graph representation (11, 11a, 11b, 13, 15a-15e, 16b-16e, 17a-17d) of the second ordered sequence is augmented by adding an additional segment (12a, 12b) corresponding to the respective segment (12a, 12b) of the i-th graph representation (11, 11a, 11b, 13, 15a-15e, 16b-16e, 17a-17d) of the second ordered sequence to the (i+1)-th graph representation (11, 11a, 11b, 13, 15a-15e, 16b-16e, 17a-17d) of the second ordered sequence;

- the at least one aggregated representation is generated depending on the augmented N2-th graph representation (11, 11a, 11b, 13, 15a-15e, 16b-16e, 17a-17d) of the second ordered sequence.

6. Computer-implemented method according to claim 5, wherein for each segment (12a, 12b) of the N1-th graph representation (11, 11a, 11b, 13, 15a-15e, 16b-16e, 17a-17d) of the first ordered sequence

v) it is determined whether the N2-th graph representation (11, 11a, 11b, 13, 15a-15e, 16b-16e, 17a-17d) of the second ordered sequence comprises a segment (12a, 12b) corresponding to the respective segment (12a, 12b) of the N1-th graph representation (11, 11a, 11b, 13, 15a-15e, 16b-16e, 17a-17d) of the first ordered sequence;
vi) if it is found that the N2-th graph representation (11, 11a, 11b, 13, 15a-15e, 17a-17d) of the second ordered sequence does not comprise a segment (12a, 12b) corresponding to the respective segment (12a, 12b) of the N1-th graph representation (11, 11a, 11b, 13, 15a-15e, 16b-16e, 17a-17d) of the first ordered sequence, the N2-th graph representation (11, 11a, 11b, 13, 15a-15e, 16b-16e, 17a-17d) of the second ordered sequence is augmented by adding an additional segment (12a, 12b) corresponding to the respective segment (12a, 12b) of the N1-th graph representation (11, 11a, 11b, 13, 15a-15e, 16b-16e, 17a-17d) of the first ordered sequence to the N2-th graph representation (11, 11a, 11b, 13, 15a-15e, 16b-16e, 17a-17d) of the second ordered sequence;

- the at least one aggregated representation is generated depending on the augmented N2-th graph representation (11, 11a, 11b, 13, 15a-15e, 16b-16e, 17a-17d) of the second ordered sequence.

7. Computer-implemented method according to one of claims 5 or 6, wherein the N1-1 iterations and the N2-1 further iterations are at least partially carried out in parallel.

8. Computer-implemented method according to one of the preceding claims, wherein

- the first graph representation (11, 11a, 11b, 13, 15a-15e, 16b-16e, 17a-17d) is registered to the second graph representation (11, 11a, 11b, 13, 15a-15e, 16b-16e, 17a-17d);
- a predefined distance metric between the registered respective segment (12a, 12b) of the first graph representation (11, 11a, 11b, 13, 15a-15e, 16b-16e, 17a-17d) and the second graph representation (11, 11a, 11b, 13, 15a-15e, 16b-16e, 17a-17d) is computed; and
- it is determined whether the second graph representation (11, 11a, 11b, 13, 15a-15e, 16b-16e, 17a-17d) comprises a segment (12a, 12b) corresponding to the respective segment (12a, 12b) of the first graph representation (11, 11a, 11b, 13, 15a-15e, 16b-16e, 17a-17d) depending on the computed distance metric.

9. Computer-implemented method according to one of the preceding claims, wherein

- a deformation field between the first graph representation (11, 11a, 11b, 13, 15a-15e, 16b-16e, 17a-17d) and the second graph representation (11, 11a, 11b, 13, 15a-15e, 16b-16e, 17a-17d) is determined; and
- the second graph representation (11, 11a, 11b, 13, 15a-15e, 16b-16e, 17a-17d) is augmented by adding the additional segment (12a, 12b) to the second graph representation (11, 11a, 11b, 13, 15a-15e, 16b-16e, 17a-17d) depending on the deformation field.

10. Computer-implemented method according to one of the preceding claims, wherein each of the graph representations (11, 11a, 11b, 13, 15a-15e, 16b-16e, 17a-17d) is given by a graph representation 11.

11. Method for generating at least one aggregated representation of a vessel structure (18), wherein

- a sequence of consecutive medical images (10, 10a, 10b, 14a-14e) depicting the vessel structure (18) is generated by an imaging device;
- a respective graph representation (11, 11a, 11b, 13, 15a-15e, 16b-16e, 17a-17d) of the vessel structure (18) is generated for each of the sequence of consecutive medical images (10, 10a, 10b, 14a, 14b, 14c, 14d, 14e); and
- a computer-implemented method according to one of the preceding claims is carried out based on the graph representations (11, 11a, 11b, 13, 15a-15e, 16b-16e, 17a-17d).

12. Method according to claim 11, wherein the sequence of consecutive medical images (10, 10a, 10b, 14a-14e) is generated as a sequence of consecutive two-dimensional images.

13. Data processing system (7, 9), which is configured to carry out a computer-implemented method according to one of claims 1 to 10.

14. Medical imaging system (1) comprising an imaging device, which is configured to generate a sequence of consecutive medical images (10, 10a, 10b, 14a-14e) depicting a vessel structure (18), and a data processing system (7, 9), which is configured to generate a respective graph representation (11, 11a, 11b, 13, 15a-15e, 16b-16e, 17a-17d) of the vessel structure (18) for each of the sequence of consecutive medical images (10, 10a, 10b, 14a-14e) and to carry out a computer-implemented method according to one of claims 1 to 10 based on the graph representations (11, 11a, 11b, 13, 15a-15e, 16b-16e, 17a-17d).

15. Computer program product comprising

- instructions, which, when carried out by a data processing system (7, 9), cause the data processing system (7, 9) to carry out a computer-implemented method according to one of claims 1 to 10; and/or
- further instructions, which, when carried out by a medical imaging system (1) according to claim 14, cause the medical imaging system (1) to carry out a method according to one of claims 11 or 12.

**Amended claims in accordance with Rule 137(2) EPC.**

1. Computer-implemented method for generating at least one aggregated representation of a vessel structure (18), wherein

- graph representations (11, 11a, 11b, 13, 15a-15e, 16b-16e, 17a-17d) of the vessel structure (18) are received, each of the graph representations (11, 11a, 11b, 13, 15a-15e, 16b-16e, 17a-17d) corresponding to a respective medical image (10, 10a, 10b, 14a-14e) of a sequence of consecutive medical images (10, 10a, 10b, 14a-14e) depicting the vessel structure (18);
- each of the graph representations (11, 11a, 11b, 13, 15a-15e, 16b-16e, 17a-17d) comprises a plurality of nodes and respective spatial coordinates for each node, wherein the plurality of nodes is arranged according to at least one segment (12a, 12b) of the respective graph representation (11, 11a, 11b, 13, 15a-15e, 16b-16e, 17a-17d);
- for each segment (12a, 12b) of a first graph representation (11, 11a, 11b, 13, 15a-15e, 16b-16e, 17a-17d) of the plurality of graph representations (11, 11a, 11b, 13, 15a-15e, 16b-16e, 17a-17d),

i) it is determined whether a second graph representation (11, 11a, 11b, 13, 15a-15e, 16b-16e, 17a-17d) of the plurality of graph representations (11, 11a, 11b, 13, 15a-15e, 16b-16e, 17a-17d) comprises a segment (12a, 12b) corresponding to the respective segment (12a, 12b) of the first graph representation (11, 11a, 11b, 13, 15a-15e, 16b-16e, 17a-17d);
ii) if it is found that the second graph representation (11, 11a, 11b, 13, 15a-15e, 16b-16e, 17a-17d) does not comprise a segment (12a, 12b) corresponding to the respective segment (12a, 12b) of the first graph representation (11, 11a, 11b, 13, 15a-15e, 16b-16e, 17a-17d), the second graph representation (11, 11a, 11b, 13, 15a-15e, 16b-16e, 17a-17d) is augmented by adding an additional segment (12a, 12b) corresponding to the respective segment (12a, 12b) of the first graph representation (11, 11a, 11b, 13, 15a-15e, 16b-16e, 17a-17d) to the second graph representation (11, 11a, 11b, 13, 15a-15e, 16b-16e, 17a-17d);

- the at least one aggregated representation is generated depending on the augmented second graph representation (11, 11a, 11b, 13, 15a-15e, 16b-16e, 17a-17d).

2. Computer-implemented method according to claim 1, wherein

- the graph representations (11, 11a, 11b, 13, 15a-15e, 16b-16e, 17a-17d) are ordered into a single ordered sequence of graph representations (11, 11a, 11b, 13, 15a-15e, 16b-16e, 17a-17d);
- for each iteration of N-1 iterations, wherein the total number of graph representations (11, 11a, 11b, 13, 15a-15e, 16b-16e, 17a-17d) of the ordered sequence is N, the graph representations (11, 11a, 11b, 13, 15a-15e, 16b-16e, 17a-17d) are indexed by $i \in [1, N] \cap \mathbb{N}$, and the N-1 iterations start with i=1 and end with i=N-1, for each segment (12a, 12b) of the i-th graph representation (11, 11a, 11b, 13, 15a-15e, 16b-16e, 17a-17d) of the ordered sequence

i) it is determined whether the (i+1)-th graph representation (11, 11a, 11b, 13, 15a-15e, 16b-16e, 17a-17d) of the ordered sequence comprises a segment (12a, 12b) corresponding to the respective segment (12a, 12b) of the i-th graph representation (11, 11a, 11b, 13, 15a-15e, 16b-16e, 17a-17d) of the ordered sequence;
ii) if it is found that the (i+1)-th graph representation (11, 11a, 11b, 13, 15a-15e, 16b-16e, 17a-17d) does not comprise a segment (12a, 12b) corresponding to the respective segment (12a, 12b) of the i-th graph representation (11, 11a, 11b, 13, 15a-15e, 16b-16e, 17a-17d), the (i+1)-th graph representation (11, 11a, 11b, 13, 15a-15e, 16b-16e, 17a-17d) is augmented by adding an additional segment (12a, 12b) corresponding to the respective segment (12a, 12b) of the i-th graph representation (11, 11a, 11b, 13, 15a-15e, 16b-16e, 17a-17d) to the (i+1)-th graph representation (11, 11a, 11b, 13, 15a-15e, 16b-16e, 17a-17d);

- the at least one aggregated representation is generated depending on the augmented N-th graph representation (11, 11a, 11b, 13, 15a-15e, 16b-16e, 17a-17d) of the ordered sequence.

3. Computer-implemented method according to claim 2, wherein, after the N-1 iterations have been carried out, for each iteration of N-1 further iterations, wherein the graph representations (11, 11a, 11b, 13, 15a-15e, 16b-16e, 17a-17d) are indexed by $j \in [1, N] \cap \mathbb{N}$, and the N-1 iterations start with j=1 and end with j=N-1, for each segment (12a, 12b) of the (N-j+1)-th graph representation (11, 11a, 11b, 13, 15a-15e, 16b-16e, 17a-17d) of the ordered sequence

iii) it is determined whether the (N-j)-th graph representation (11, 11a, 11b, 13, 15a-15e, 16b-16e, 17a-17d) of the ordered sequence comprises a segment (12a, 12b) corresponding to the respective segment (12a, 12b) of the (N-j+1)-th graph representation (11, 11a, 11b, 13, 15a-15e, 16b-16e, 17a-17d) of the ordered sequence;
iv) if it is found that the (N-j)-th graph representation (11, 11a, 11b, 13, 15a-15e, 16b-16e, 17a-17d) does not comprise a segment (12a, 12b) corresponding to the respective segment (12a, 12b) of the (N-j+1)-th graph representation (11, 11a, 11b, 13, 15a-15e, 16b-16e, 17a-17d), the (N-j)-th graph representation (11, 11a, 11b, 13, 15a-15e, 16b-16e, 17a-17d) is augmented by adding an additional segment (12a, 12b) corresponding to the respective segment (12a, 12b) of the (N-j+1)-th graph representation (11, 11a, 11b, 13, 15a-15e, 16b-16e, 17a-17d) to the (N-j)-th graph representation (11, 11a, 11b, 13, 15a-15e, 16b-16e, 17a-17d);

- the at least one aggregated representation is generated depending on the augmented initial graph representation (11, 11a, 11b, 13, 15a-15e, 16b-16e, 17a-17d) of the ordered sequence.

4. Computer-implemented method according to claim 1, wherein

- the graph representations (11, 11a, 11b, 13, 15a-15e, 16b-16e, 17a-17d) are grouped into at least two ordered sequences of graph representations (11, 11a, 11b, 13, 15a-15e, 16b-16e, 17a-17d) including a first ordered sequence and a second ordered sequence;
- for each iteration of N1-1 iterations, wherein the total number of graph representations (11, 11a, 11b, 13, 15a-15e, 16b-16e, 17a-17d) of the first ordered sequence is N1, the graph representations (11, 11a, 11b, 13, 15a-15e, 16b-16e, 17a-17d) of the first ordered sequence are indexed by $i \in [1, N1] \cap \mathbb{N}$, and the N1-1 iterations start with i=1 and end with i=N1-1, for each segment (12a, 12b) of the i-th graph representation (11, 11a, 11b, 13, 15a-15e, 16b-16e, 17a-17d) of the first ordered sequence

i) it is determined whether the (i+1)-th graph representation (11, 11a, 11b, 13, 15a-15e, 16b-16e, 17a-17d) of the first ordered sequence comprises a segment (12a, 12b) corresponding to the respective segment (12a, 12b) of the i-th graph representation (11, 11a, 11b, 13, 15a-15e, 16b-16e, 17a-17d) of the first ordered sequence;
ii) if it is found that the (i+1)-th graph representation (11, 11a, 11b, 13, 15a-15e, 16b-16e, 17a-17d) of the first ordered sequence does not comprise a segment (12a, 12b) corresponding to the respective segment (12a, 12b) of the i-th graph representation (11, 11a, 11b, 13, 15a-15e, 16b-16e, 17a-17d) of the first ordered sequence, the (i+1)-th graph representation (11, 11a, 11b, 13, 15a-15e, 16b-16e, 17a-17d) of the first ordered sequence is augmented by adding an additional segment (12a, 12b) corresponding to the respective segment (12a, 12b) of the i-th graph representation (11, 11a, 11b, 13, 15a-15e, 16b-16e, 17a-17d) of the first ordered sequence to the (i+1)-th graph representation (11, 11a, 11b, 13, 15a-15e, 16b-16e, 17a-17d) of the first ordered sequence;

- the at least one aggregated representation is generated depending on the augmented N1-th graph representation (11, 11a, 11b, 13, 15a-15e, 16b-16e, 17a-17d) of the first ordered sequence.

5. Computer-implemented method according to claim 4, wherein

- for each iteration of N2-1 further iterations, wherein the total number of graph representations (11, 11a, 11b, 13, 15a-15e, 16b-16e, 17a-17d) of the second ordered sequence is N2, the graph representations (11, 11a, 11b, 13, 15a-15e, 16b-16e, 17a-17d) of the second ordered sequence are indexed by $i \in [1, N2] \cap \mathbb{N}$, and the N2-1 further iterations start with i=1 and end with i=N2-1, for each segment (12a, 12b) of the i-th graph representation (11, 11a, 11b, 13, 15a-15e, 16b-16e, 17a-17d) of the second ordered sequence

iii) it is determined whether the (i+1)-th graph representation (11, 11a, 11b, 13, 15a-15e, 16b-16e, 17a-17d) of the second ordered sequence comprises a segment (12a, 12b) corresponding to the respective segment (12a, 12b) of the i-th graph representation (11, 11a, 11b, 13, 15a-15e, 16b-16e, 17a-17d) of the second ordered sequence;
iv) if it is found that the (i+1)-th graph representation (11, 11a, 11b, 13, 15a-15e, 16b-16e, 17a-17d) of the second ordered sequence does not comprise a segment (12a, 12b) corresponding to the respective segment (12a, 12b) of the i-th graph representation (11, 11a, 11b, 13, 15a-15e, 16b-16e, 17a-17d) of the second ordered sequence, the (i+1)-th graph representation (11, 11a, 11b, 13, 15a-15e, 16b-16e, 17a-17d) of the second ordered sequence is augmented by adding an additional segment (12a, 12b) corresponding to the respective segment (12a, 12b) of the i-th graph representation (11, 11a, 11b, 13, 15a-15e, 16b-16e, 17a-17d) of the second ordered sequence to the (i+1)-th graph representation (11, 11a, 11b, 13, 15a-15e, 16b-16e, 17a-17d) of the second ordered sequence;

- the at least one aggregated representation is generated depending on the augmented N2-th graph representation (11, 11a, 11b, 13, 15a-15e, 16b-16e, 17a-17d) of the second ordered sequence.

**6.** Computer-implemented method according to claim 5, wherein for each segment (12a, 12b) of the N1-th graph representation (11, 11a, 11b, 13, 15a-15e, 16b-16e, 17a-17d) of the first ordered sequence

> v) it is determined whether the N2-th graph representation (11, 11a, 11b, 13, 15a-15e, 16b-16e, 17a-17d) of the second ordered sequence comprises a segment (12a, 12b) corresponding to the respective segment (12a, 12b) of the N1-th graph representation (11, 11a, 11b, 13, 15a-15e, 16b-16e, 17a-17d) of the first ordered sequence;
> vi) if it is found that the N2-th graph representation (11, 11a, 11b, 13, 15a-15e, 16b-16e, 17a-17d) of the second ordered sequence does not comprise a segment (12a, 12b) corresponding to the respective segment (12a, 12b) of the N1-th graph representation (11, 11a, 11b, 13, 15a-15e, 16b-16e, 17a-17d) of the first ordered sequence, the N2-th graph representation (11, 11a, 11b, 13, 15a-15e, 16b-16e, 17a-17d) of the second ordered sequence is augmented by adding an additional segment (12a, 12b) corresponding to the respective segment (12a, 12b) of the N1-th graph representation (11, 11a, 11b, 13, 15a-15e, 16b-16e, 17a-17d) of the first ordered sequence to the N2-th graph representation (11, 11a, 11b, 13, 15a-15e, 16b-16e, 17a-17d) of the second ordered sequence;

> - the at least one aggregated representation is generated depending on the augmented N2-th graph representation (11, 11a, 11b, 13, 15a-15e, 16b-16e, 17a-17d) of the second ordered sequence.

**7.** Computer-implemented method according to one of claims 5 or 6, wherein the N1-1 iterations and the N2-1 further iterations are at least partially carried out in parallel.

**8.** Computer-implemented method according to one of the preceding claims, wherein

> - the first graph representation (11, 11a, 11b, 13, 15a-15e, 16b-16e, 17a-17d) is registered to the second graph representation (11, 11a, 11b, 13, 15a-15e, 16b-16e, 17a-17d);
> - a predefined distance metric between the registered respective segment (12a, 12b) of the first graph representation (11, 11a, 11b, 13, 15a-15e, 16b-16e, 17a-17d) and the second graph representation (11, 11a, 11b, 13, 15a-15e, 16b-16e, 17a-17d) is computed; and
- it is determined whether the second graph representation (11, 11a, 11b, 13, 15a-15e, 16b-16e, 17a-17d) comprises a segment (12a, 12b) corresponding to the respective segment (12a, 12b) of the first graph representation (11, 11a, 11b, 13, 15a-15e, 16b-16e, 17a-17d) depending on the computed distance metric.

**9.** Computer-implemented method according to one of the preceding claims, wherein

> - a deformation field between the first graph representation (11, 11a, 11b, 13, 15a-15e, 16b-16e, 17a-17d) and the second graph representation (11, 11a, 11b, 13, 15a-15e, 16b-16e, 17a-17d) is determined; and
> - the second graph representation (11, 11a, 11b, 13, 15a-15e, 16b-16e, 17a-17d) is augmented by adding the additional segment (12a, 12b) to the second graph representation (11, 11a, 11b, 13, 15a-15e, 16b-16e, 17a-17d) depending on the deformation field.

**10.** Computer-implemented method according to one of the preceding claims, wherein each of the graph representations (11, 11a, 11b, 13, 15a-15e, 16b-16e, 17a-17d) is given by a rooted graph representation (11).

**11.** Method for generating at least one aggregated representation of a vessel structure (18), wherein

> - a sequence of consecutive medical images (10, 10a, 10b, 14a-14e) depicting the vessel structure (18) is generated by an imaging device;
> - a respective graph representation (11, 11a, 11b, 13, 15a-15e, 16b-16e, 17a-17d) of the vessel structure (18) is generated for each of the sequence of consecutive medical images (10, 10a, 10b, 14a, 14b, 14c, 14d, 14e); and
> - a computer-implemented method according to one of the preceding claims is carried out based on the graph representations (11, 11a, 11b, 13, 15a-15e, 16b-16e, 17a-17d).

**12.** Method according to claim 11, wherein the sequence of consecutive medical images (10, 10a, 10b, 14a-14e) is generated as a sequence of consecutive two-dimensional images.

**13.** Data processing system (7, 9), which is configured to carry out a computer-implemented method according to one of claims 1 to 10.

**14.** Medical imaging system (1) comprising an imaging device, which is configured to generate a sequence of consecutive medical images (10, 10a, 10b, 14a-14e) depicting a vessel structure (18), and a data processing system (7, 9) according to claim 13, wherein the data processing system (7, 9) is configured to generate a respective graph representation (11, 11a, 11b, 13, 15a-15e, 16b-16e, 17a-17d) of the vessel structure (18) for each of the sequence of consecutive medical images (10, 10a, 10b, 14a-14e) and to carry out the computer-implemented method according to one of claims 1 to 10 based on the graph representations (11, 11a, 11b, 13, 15a-15e, 16b-16e, 17a-17d).

**15.** Computer program product comprising

- instructions, which, when carried out by a data processing system (7, 9), cause the data processing system (7, 9) to carry out a computer-implemented method according to one of claims 1 to 10; and/or
- further instructions, which, when carried out by a medical imaging system (1) according to claim 14, cause the medical imaging system (1) to carry out a method according to one of claims 11 or 12.

FIG 1

FIG 2

FIG 3

FIG 4

14a, 15a
14b, 15b, 16b
14c, 15c, 16c
14d, 15d, 16d
14e, 15e, 16e

# FIG 5

## FIG 6

Europäisches
Patentamt

European
Patent Office

Office européen
des brevets

# EUROPEAN SEARCH REPORT

**Application Number**

EP 24 46 5529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/110874 A1 (MATTHEWS JAMES [GB] ET AL) 21 April 2016 (2016-04-21) <br> * abstract * <br> * figures 2,3,8-10 * <br> * paragraphs [0001], [0032], [0042], [0046], [0051], [0052], [0093] – [0096], [0103], [0147] * <br> ----- | 1-15 | INV. <br> G06T7/13 <br> G06T7/162 |
| | | | **TECHNICAL FIELDS SEARCHED (IPC)** <br><br> G06T |

The present search report has been drawn up for all claims

| Place of search | Date of completion of the search | Examiner |
|---|---|---|
| Munich | 19 November 2024 | Scholz, Volker |

EPO FORM 1503 03.82 (P04C01)

## ANNEX TO THE EUROPEAN SEARCH REPORT
## ON EUROPEAN PATENT APPLICATION NO.

EP 24 46 5529

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2024

| Patent document cited in search report | Publication date | Patent family member(s) | | Publication date |
|---|---|---|---|---|
| US 2016110874 A1 | 21-04-2016 | JP | 6632860 B2 | 22-01-2020 |
| | | JP | 2016077906 A | 16-05-2016 |
| | | US | 2016110874 A1 | 21-04-2016 |

EPO FORM P0459

For more details about this annex : see Official Journal of the European Patent Office, No. 12/82